# FASCICULE DE BREVET EUROPEEN

(11) **EP 0 785 924 B1**
(45) Date de publication et mention de la délivrance du brevet: **03.05.2000**
(21) Numéro de dépôt: 95935490.3
(22) Date de dépôt: 13.10.1995
(51) Int. Cl.: C07D 213/73, A61K 31/44

(54) **BIS-2-AMINOPYRIDINES, LEUR PROCEDE DE PREPARATION ET LEURS APPLICATIONS CONTRE LES PARASITOSES**
BIS-2-AMINOPYRIDINE, VERFAHREN ZU IHRER HERSTELUNG UND IHRE VERWENDUNG ZUR BEKÄMPFUNG VON PARASITOSEN
BIS-2-AMINOPYRIDINES, PREPARATION METHOD THEREFOR AND USE THEREOF FOR CONTROLLING PARASITIC INFECTIONS

(30) Priorité: 14.10.1994 FR 9412301
(43) Date de publication de la demande: 30.07.1997
(73) Titulaire: VIRBAC S.A., F-06516 Carros Cedex (FR)
(72) Inventeur: VIAL, Henri, F-34080 Montpellier (FR); CALAS, Michèle, F-34090 Montpellier (FR); BOURGUIGNON, Jean-Jacques, F-67150 Hipsheim (FR); ANCELIN, Marie-Laure, F-34270 Saint-Jean-de-Cuculles (FR); GIRAL, Louis, F-34000 Montpellier (FR)
(74) Mandataire: Ores, Irène
(86) Numéro de dépôt international: FR9501349
(87) Numéro de publication internationale: WO9611910

(56) Documents cités:
- EP-A- 0 494 754

## Description

La présente invention est relative à des bis-2-aminopyridines substituées, à leur procédé de préparation ainsi qu'aux applications des bis-2-aminopyridines substituées ou non, comme médicament, notamment comme médicaments actifs sur les parasitoses intra-érythrocytaires, telles que le paludisme ou la babésiose (ou piroplasmose).

On entend par activité antipaludéenne et antibabésiose, la capacité d'empêcher le développement du parasite à l'intérieur de l'érythrocyte et/ou l'invasion érythrocytaire et d'entraîner la mort des parasites initialement présents.

Le paludisme ou malaria reste la plus importante des maladies parasitaires qui sévit dans les zones intertropicales. L'Organisation Mondiale de la Santé estime qu'il y a dans le monde quelque 350 millions de cas de paludisme, dont 90 % en Afrique, causant la mort de 1,5 à 2,7 millions de personnes par an. Dès 1940, les antifolates de synthèse comme Fansidar® (sulfonamide pyriméthamine), les quinolines comme Nivaquine® (chloroquine) étaient employés pour combattre la maladie. Cependant, dès les années 1960, des résistances sont apparues et ont nécessité la mise au point de nouvelles molécules actives, parmi lesquelles le Lariam® (méfloquine) ou l'Halfan® (halofantrine) ; toutefois, ces nouvelles molécules ont également conduit à l'émergence de nouvelles résistances, résistances spécifiques mais aussi résistances croisées. L'émergence de souches de *Plasmodium falciparum* chloroquino-résistantes en Asie du Sud-est et en Amérique latine, et l'expansion générale de ces pharmaco-résistances apportent une limite au traitement efficace du paludisme. En outre, la résistance à chacun des nouveaux composés, méfloquine et halofantrine est également importante et fait apparaître une résistance croisée entre ces deux substances.

D'autres substances telles que l'artémisinine et ses dérivés (artemether, artether, artesunate, et dihydroartemisinine) sont également utilisées en Chine et en Asie du Sud-est. Cependant, ces substances ont l'inconvénient de présenter une demi-vie courte et une faible hydrosolubilité.

Les babésioses, encore appelées piroplasmoses, sont comme la malaria, des parasitoses intra-érythrocytaires transmises par les morsures de tiques, qui affectent de nombreuses espèces animales domestiques et sauvages et dont l'agent est un protozoaire du genre *Babesia.* Il existe ainsi une babésiose bovine causée par *Babesia bovis, Babesia bigemina, Babesia major* et *Babesia divergens* responsables de pertes importantes dans l'élevage bovin, notamment dans les pays en voie de développement. On note aussi une babésiose canine avec *Babesia canis* et *Babesia gibsoni,* une babésiose équine avec *Babesia equi* et *cabalii* et même une babésiose humaine, rare mais sévère, due à *Babesia divergens* et *Babesia microti.*

Peu de substances sont actives contre *Babesia* et sont généralement toxiques (imidocarb et amicarbalide). D'autres produits se révèlent actifs *in vitro* sans pour autant donner de bons résultats *in vivo* comme la tubercidine, la tétracycline ou la ménoctone.

Dès 1984, Mc Colm, (Ann. Trop. Med. Parasitol., 78, (4), 345) a souligné la résistance accrue de *Babesia,* vis-à-vis des antimicrobiens classiques tels que tylosin, rifamycine, gramicidine D.

En conséquence, la Demanderessse s'est donné pour but de pourvoir à de nouveaux produits actifs sur les parasites intra-érythrocytaires.

La présente invention a pour objet des bis-2-aminopyridine substituées de formule générale (I), 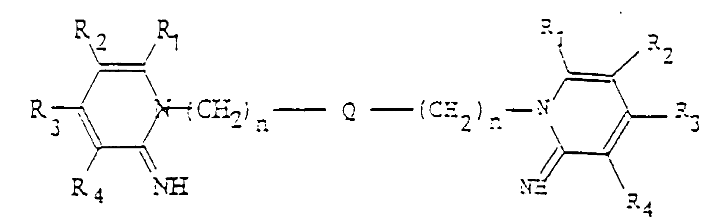 dans laquelle :
Q représente :
   - un groupement alkyle, saturé ou insaturé, en C₆-C₂₀,
   - un groupement aryle, éventuellement substitué, ou
   - un groupement cycloalkyle, et
   forme, entre les 2 noyaux pyridine et avec les groupements (CH₂)ₙ, un enchaînement hydrocarboné comprenant au total de 6 à 34 atomes de carbone,
n est un nombre compris entre 0 et 7,
R₁, R₂, R₃, R₄, identiques ou différents, représentent :
   - un atome hydrogène,
   - un groupement alkyle en C₁-C₆, linéaire ou ramifié, éventuellement substitué,
   - un groupement aryle, éventuellement substitué par un ou plusieurs atomes d'halogène, ou des radicaux alkyle en C₁-C₆ ou alcoxy en C₁-C₆,
   - un groupement benzyle,
   - un groupement thiényle, de préférence un -2-thiényle,
   - un groupement furyle, de préférence un -2-furyle,
   - un atome d'halogène,
   - un groupement alcoxy ou benzyloxy
   - un groupement trifluorométhyle,
   - un groupement vinyle ou allyle,
à la condition qu'au moins l'un des radicaux R₁, R₂, R₃ ou R₄ ne soit pas un atome d'hydrogène, lorsque Q représente un enchaînement (CH₂)ₘ avec m compris entre 6 et 20, ainsi que leurs sels d'addition.

Parmi les sels d'addition avec les acides, on peut citer ceux formés avec les acides minéraux tels que les acides chlorhydrique, bromhydrique, nitrique, sulfurique ou phosphorique ou les acides organiques tels que les acides acétique, propionique, maléique, benzoïque, succinique, méthanesulfonique, paratoluènesulfonique, fumarique, hydroxyéthanesulfonique.

L'expression alkyle en C₆-C₂₀ désigne des groupements alkyles, saturés ou insaturés, linéaires ou ramifiés.

L'expression aryle désigne par exemple, un radical phényle, éventuellement substitué par un ou plusieurs atomes de fluor, de chlore ou de brome ou des radicaux alkyles ou alcoxy.

L'expression cycloalkyle peut désigner par exemple, un radical cyclopropyle, cyclobutyle, cylcopentyle, cyclohexyle.

L'expression alkyle en C₁-C₅ désigne des groupements alkyles, saturés ou insaturés, linéaires ou ramifiés, comme par exemple les groupements méthyle, éthyle, propyle, butyle, isopropyle, isobutyle, sec-butyle, tertiobutyle, pentyle, hexyle.

L'expression alcoxy désigne des groupements alcoxy en C₁-C₆ tels que les groupements méthoxy, éthoxy, propoxy, butoxy.

L'expression halogène désigne un atome de fluor, de chlore ou de brome.

Bien que, d'une part, des 3-hydroxypyridin-4-one à activité antiparasitaire soient connues (Demande de Brevet européen 0 494 754) et que, d'autre part, d'autres bis-aminopyridines aient été décrites : bis amino-4-pyridines (Brevet US 4 206 215 et J. Med. Chem. 1984, 27, 1457-1464), qui présentent des activités sur le traitement de la plaque dentaire ou des bis amino-2-pyridines non substituées sur les cycles pyridines ayant une activité de catalyseurs d'iodation (INOKUMA, Yakagaku, 1982, 31, (8), 515-19), la Demanderesse a trouvé de façon surprenante qu'aussi bien les bis amino-2-pyridines non substituées que les bis amino-2-pyridines portant au minimum une subtitution, telles que définies ci-dessus, possèdent une activité antiparasitaire et notamment une activité antipaludique et antibabésiose, tout à fait intéressante et inattendue, les bis-amino-2-pyridines substituées présentent une activité particulièrement intéressante.

En effet, les produits de formule I tels que définis ci-dessus, ainsi que les produits de formule I dans lesquels R₁, R₂, R₃ et R₄ représentent des atomes d'hydrogène, ainsi que leurs sels d'addition avec les acides, présentent d'intéressantes propriétés pharmacologiques. Ils sont doués, en particulier, d'une très bonne activité contre les parasitoses intra-érythrocytaires.

De manière inattendue, ces produits bloquent la prolifération érythrocytaire du parasite. En effet, ces produits interfèrent dans le métabolisme du parasite, en bloquant le transport de la choline, précurseur indispensable des phosphatidylcholines, constituants des membranes cellulaires et nécessaires à la prolifération des parasites, laquelle s'accompagne d'une néosynthèse considérable des phospholipides nécessaires à la biogénèse des membranes cellulaires.

Les composés selon l'invention présentent une très forte activité *in vitro* contre des souches ou des isolats diversement chimiorésistants. L'activité s'exerce principalement contre les stades érythrocytaires matures, phase la plus intense de synthèse des phospholipides. En particulier, dans les systèmes murins infectés par *Plasmodium* *chabaudi ou Plasmodium petteri*, les composés selon l'invention éradiquent complètement la parasitémie.

L'invention a plus particulièrement pour objet les produits définis ci-dessus, caractérisés en ce que dans la formule (I), **R**_{**1**}, **R**_{**2**}, **R**_{**3**} ou **R**_{**4**} représentent un groupe méthyle, chloro, phényle, en positions 3, 4 et/ou 5 et **Q** représente un groupement alkyle en C₆-C₂₀, l'enchaînement hydrocarboné entre les deux noyau pyridine constituant un dodécane, un hexadécane, un octane ou un décane.

Selon l'invention, les produits de formule I ci-dessus et leurs sels peuvent être préparés par un procédé caractérisé en ce que l'on fait réagir, en solution, un dérivé de formule (II) Hal-(CH₂)ₙ-Q-(CH₂)ₙ-Hal, dans laquelle Hal représente un atome d'halogène, n et Q ont la signification générale indiquée précédemment, avec un composé de formule (III) : 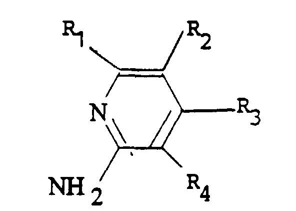 dans laquelle R₁, R₂, R₃ et R₄ ont la signification donnée précédemment,
pour obtenir le produit de formule générale (I), à l'état de dihalogénure correspondant que, si désiré, l'on basifie et l'on convertit en un autre sel d'acide.

Dans les conditions préférentielles de mise en oeuvre de l'invention, le procédé ci-dessus décrit est réalisé de la manière suivante :

La réaction d'un composé de formule II et d'un composé de formule III, présents dans un rapport 1:2, le composé de formule II étant de préférence en léger excès, pour aboutir à la conversion totale des aminopyridines (dérivé de formule III), est réalisée :
. en présence d'un solvant ou d'un mélange de solvants inertes vis-à-vis des réactifs mis en oeuvre, choisi parmi l'acétonitrile, les hydrocarbures aromatiques (toluène, xylènes), les cétones aliphatiques comme la propanone, la butanone, la -3-méthyl-2-butanone, les alcools (méthanol, éthanol, propanol, butanol), le diméthylformamide, les éthers courants par exemple le dioxane, le monoglyme, le diglyme,
. à température ambiante ou à chaud (température d'ébullition du solvant, notamment),
. pendant quelques heures à plusieurs jours.

La réaction peut être également menée sans ajout de solvant, dans la mesure où l'un des réactifs est liquide à la température de réaction et dissout ainsi l'autre réactif. Un avantage du présent procédé est d'obtenir directement en fin de réaction le dihalogénohydrate, en général bromhydrate ou chlorhydrate des bis-aminopyridines recherchées qui cristallise directement à l'état pur dans le solvant.

On peut toutefois obtenir les bases correspondantes par déplacement de l'ion halogénohydrique, par exemple avec une résine échangeuse d'ions ou par précipitation d'un halogénure d'argent et les convertir en un autre sel d'acide minéral ou organique pharmaceutiquement acceptable.

L'ensemble des produits de formule (I) et leurs sels d'acides acceptables du point de vue pharmaceutique peuvent être administrés à l'homme ou à l'animal en tant que médicament, sous la forme de compositions pharmaceutiques comprenant comme constituant actif, une dose efficace d'au moins un produit de formule (I) ou d'un sel d'addition d'acide d'un tel composé avec, en plus des excipients et additifs, pris parmi ceux dont on se sert ordinairement en pharmacie.

Ces compositions peuvent être administrées par voie orale, parentérale ou rectale, notamment sous la forme de comprimés, dragées, capsules, solutions, sirops, émulsions ou suspensions, formes galéniques capables de moduler la libération de la substance active.

De telles compositions sont généralement administrées à la dose de 0,5 à 50 mg/kg.

Outre les dispositions qui précèdent, l'invention comprend encore d'autres dispositions, qui ressortiront de la description qui va suivre, qui se réfère à des exemples de mise en oeuvre du procédé objet de la présente invention.

Il doit être bien entendu, toutefois, que ces exemples sont donnés uniquement à titre d'illustration de l'objet de l'invention, dont ils ne constituent en aucune manière une limitation.

Tous les dérivés synthétisés ont été soumis à une analyse élémentaire, avec une tolérance maximale de 0,3 % dans les résultats obtenus, par rapport aux résultats calculés.

Les spectres de résonance magnétique nucléaire du proton ont été réalisés en solution dans CDCl3 + TMS ou dans le DMSO D₆ sur un appareil Brucker WP 200. Les déplacements chimiques les plus caractéristiques sont éventuellement indiqués dans ces exemples. Sauf indication particulière, tous les produits ont été isolés à l'état de bromhydrates.

### EXEMPLE 1 : 1,1-(1,12-dodécanediyl) bis-(4-méthyl-2-(1H) pyridinimine) dibrombydrate.

On dissout 0,1 Mole de 1,12-dibromododécane commercial dans 500 millilitres de méthyléthylcétone, on introduit sous argon 0,15 mole de 4-méthyl-2-aminopyridine en solution dans la méthyléthylcétone et laisse agiter à température du reflux jusqu'à accomplissement de la réaction que l'on peut suivre par chromatographie en couche mince, le plus généralement 48 heures. Après filtration et rinçage du précipité, on obtient le produit brut sous forme de son bromhydrate 1. Le produit peut être recristallisé dans un mélange iso-propanol/méthanol 9/1. On obtient ainsi de beaux cristaux blancs de pF = 206°C avec un rendement sur le produit pur de 66%.

En variante, le produit est préparé comme suit :
0,36 g (3,3 mmoles) de 4-méthyl-2-aminopyridine sont dissous dans 5 ml de n-BuOH, et mis à reflux sous argon pendant 48 heures en présence de 0,50 g (1,52 mmoles) de 1,12-dibromododécane. Après refroidissement du milieu réactionnel, le produit critallise.

Il est récupéré par filtration, rincé avec un peu d'isopropanol, puis de l'éther et séché.

On obtient 0,30 g de sel (rendement 47 %).

Formule brute : C₂₄H₄₀N₄Br₂ ; poids moléculaire : 436,28; RMN¹H (200 MHz, DMSO-D₆): 8,27 (S large, 4H, 2x-NH₂ échangeables); 7,98-7,96 (partie A d'un système AB, 2H, 12xH₆); 6,81 (m, 4H, 2x(H₃+H₅)); 4,08-4,05 (m, 4H, 2x>N-CH₂-); 2,31 (s, 6H, 2x-CH₃) ; 1,23-1,63 (m, 20H, 10x-CH₂-).

### EXEMPLE 2 : 1,1-(1,12-dodécanediyl) bis-(3-méthyl-2-(1H) pyridinimine) dibromhydrate.

On dissout 0,1 Mole de 1,12-dibromododécane commercial dans 500 millilitres de méthyléthylcétone, on introduit sous argon 0,15 mole de 3-méthyl-2-aminopyridine en solution dans la méthyléthylcétone et laisse agiter à température du reflux jusqu'à accomplissement de la réaction que l'on peut suivre par chromatographie en couche mince, le plus généralement 48 heures. Après filtration et rinçage du précipité, on obtient le produit brut sous forme de son bromhydrate . Le produit peut être recristallisé dans un mélange iso-propanol/méthanol 9/1. On obtient ainsi de beaux cristaux blancs de pF = 229°C avec un rendement sur le produit pur de 38 %.

Formule brute : C₂₄H₄₀N₄Br₂ ; poids moléculaire : 544,42 ; RMN¹H (200 MHz, DMSO-D₆) : 8,08 (S large, 4H, 2x-NH₂ échangeables) ; 8,00 (partie A d'un système AB, 2H, 2xH₆) ; 7,77 (m, 2H, 2xH₅) 6,88-6,85 (partie B d'un système AB, 2H, 2xH₄) ; 4,21 (m, 4H, 2x>N-CH₂-); 2,22 (s, 6H, 2x-CH₃) ; 1,23-1,63 (m, 20H, 10x-CH₂-).

### EXEMPLE 3 : 1,1-(1,12-dodécanediyl) bis-(5-méthyl-2-(1H) pyridinimine) dibromhydrate.

On dissout 0.1 Mole de 1,12-dibromododécane commercial dans 500 millilitres de méthyléthylcétone, on introduit sous argon 0,15 mole de 5-méthyl-2-aminopyridine en solution dans la méthyléthylcétone et laisse agiter à température du reflux jusqu'à accomplissement de la réaction que l'on peut suivre par chromatographie en couche mince. Après filtration et rinçage du précipité, on obtient le produit brut sous forme de son bromhydrate. Le produit peut être recristallisé dans un mélange iso-propanol/méthanol 9/1. On obtient le dérivé du titre avec un rendement de 40 % présentant un pF = 221°C.

Formule brute : C₂₄H₄₀N₄Br₂ ; poids moléculaire : 544,42 ; RMN¹H (200 MHz, DMSO-D₆) : 8,08 (S large, 4H, 2x-NH₂ échangeables) ; 8,00 (m, 2H, 2xH₆) ; 7,70 (partie A d'un système AB, 2H, 2xH₃) ; 6,80 (partie B d'un système AB, 2H, 2xH₄) ; 4,20 (m, 4H, 2x>N-CH₂-); 1,2-1,63 (m, 20H, 10x-CH₂-).

### EXEMPLE 4 : 1,1-(1,12-dodécanediyl) bis-(6-méthyl-2-(1H) pyridinimine) dibromhydrate.

On opère dans les conditions décrites ci-dessus à partir de -6-méthyl-2-aminopyridine. Le produit est purifié par recristallisation dans l'isopropanol. On obtient le dérivé du titre de pF = 205°C avec un rendement de 23 %.

### EXEMPLE 5 : 1,1-(1,12-dodécanediyl) bis-(5-phényl-2-(1H) pyridinimine) dibromhydrate.

On opère comme précédemment avec du dibromododécane et de la 2-amino-5-phényl-pyridine. Le produit obtenu est purifié par recristallisation dans le méthanol. Son pf = 185°C.

### EXEMPLE 6 : 1,1-(1,12-dodécanediyl) bis-(3-phénylméthoxy-2-(1H) pyridinimine) dibromhydrate.

On opère comme ci-dessus avec du dibromododécane et de la 2-amino-3-phénylméthoxy-pyridine qui a été synthétisée par la méthode décrite par Rydzkowski R. dans Tetrahedron Letters 26 (1985) 2571-2574.

### Exemple 7 : 1,1-(1,12-dodécanediyl) bis-(4-(4-méthylphényl)-2-(1H) pyridinimine) dibromhydrate.

On opère comme ci-dessus avec du dibromododécane et de la 2-amino-4-paratolyl-pyridine, qui est synthétisée selon Chambron J.Cl. Strasbourg Tetrah. 1987 43 (5) 895/905. Le produit obtenu est purifié à l'état de base sur colonne de silice par un mélange benzène-acétate d'éthyle. On le retransforme en bromhydrate, par un mélange isopropanol bromhydrique/éther. On peut alors le recristalliser.

### EXEMPLE 8 : 1,1-(1,12-dodécanediyl) bis-(3-n-butyl-2-(1H) pyridinimine) dibromhydrate.

On prépare la 2-amino-3-n-butyl pyridine selon Gassman J. Amer. Chem. Soc. 95 (1973) 4453 et condense comme ci-dessus sur le dibromododécane correspondant. On obtient le produit recherché avec un pF = 192°C.

### EXEMPLE 9 : 1,1-(1,12-dodécanediyl) bis-(-5-chloro-2-(1H) pyridinimine) dibromhydrate.

On opère dans les conditions de l'exemple 1 avec la 2-amino-5-chloro-pyridine dans la méthylethylcétone. Après recristallisations dans le mélange isopropanolméthanol 9/1 on obtient le dérivé du titre de pF = 239°C avec un rendement de 18 %.

Ce produit présente la formule brute suivante : C₂₂H₃₄N₄Br₂Cl₂.

Poids moléculaire : 585,26.

RMN¹H (200 MHz, DMSO-d₆) : 8,67 (S large, 4H, 2x-NH₂ + échangeables) ; 8,46 (m, 2H, 2xH₆) ; 7,99 (partie A d'un système AB, 2H, 2xH₃) ; 7,05 (partie B d'un système AB, 2H, 2xH₄) ; 4,09 (m, 4H, 2x >N-CH₂-) ; 1,2-1,66 (m, 20H, 10x-CH₂-).

### EXEMPLE 10 : 1,1-(1,12-dodécanediyl) bis-(-4-bromo-2-(1H) pyridinimine) dibromhydrate.

On opère comme ci-dessus en utilisant la 2-amino-4-bromopyridine (selon Larsen, Scott WO 93/25553) et l'on obtient le dérivé recherché avec un pF = 230°C.

### EXEMPLE 11 : 1,1-(1,12-dodécanediyl) bis (-3-chloro-5-trifluorométhyl-2-(1H) pyridinimine) dibromhydrate.

On utilise la 2-amino-3-chloro-5-trifluorométhylpyridine synthétisée selon Haga Takahiro Heterocycles, 22 (1984) 1, 117-124. En traitant selon le procédé précédent, on isole le dérivé de pF = 185°C.

### EXEMPLE 12 : 1,1-(1,8-octanediyl) bis-(5-méthyl-2-(1H) pyridinimine) dibrombydrate.

On dissout 0,1 Mole de 1,8-dibromooctane commercial dans 500 millilitres de méthyléthylcétone, on introduit sous argon 0,15 mole de 5-méthyl-2-aminopyridine en solution dans la méthyléthylcétone et laisse agiter à température du reflux jusqu'à accomplissement de la réaction que l'on peut suivre par chromatographie en couche mince, le plus généralement 48 heures. Après filtration et rinçage du précipité, on obtient le produit brut sous forme de son bromhydrate. Le produit peut être recristallisé dans un mélange iso-propanol/méthanol 9/1. On obtient le dérivé du titre avec un rendement de 40 % présentant un pF = 241°C.

### EXEMPLE 13 : 1,1-(1,8-octanediyl) bis-(4-méthyl-2-(1H) pyridinimine) dibromhydrate.

On dissout 0,1 Mole de 1,8-dibromooctane commercial dans 500 millilitres de méthyléthylcétone, on introduit sous argon 0,15 mole de 4-méthyl-2-aminopyridine en solution dans la méthyléthylcétone et laisse agiter à température du reflux jusqu'à accomplissement de la réaction que l'on peut suivre par C.C.M.. Après filtration et rinçage du précipité, on obtient le produit brut sous forme de son bromhydrate. Le produit peut être recristallisé dans un mélange iso-propanol/méthanol 9/1. On obtient ainsi de beaux cristaux blancs de pF = 248°C avec un rendement sur le produit pur de 47%.

En variante, ce produit est préparé comme suit :

Dans un erlenmeyer rodé de 100 ml muni d'un réfrigérant et d'un agitateur magnétique, on introduit 2 g de 2-amino-4-méthylpyridine (18,5 mmoles, 3 équivalents) et 6,16 mmoles (1,7 ml) de 1,8-dibromooctane dans 50 ml de butanone. Le mélange est chauffé à reflux 36 heures, puis est laissé à 4°C une nuit. Le précipité est filtré, recristallisé dans un mélange méthanol/isopropanol/éther (48 h à 4°C). On obtient ainsi de beaux cristaux blancs de pF compris entre 247-250°C avec un rendement sur le produit pur de 52 %.

RMN¹H dans DMSO-D₆ (δ en ppm) : 1,25 (m, 8H) ; 1,65 (m, 4H) ; 2,3 (s, 6H) ; 4,1 (t, 4H) ; 6,85 (m, 4H) ; 8,00 (d, 2H) ; 8,45 (s, 4H).

### EXEMPLE 14 : 1,1-(1,6-hexanediyl) bis-(4-méthyl-2-(1H) pyrimidine) dibromhydrate.

On opère dans les conditions décrites ci-dessus avec du 1,6-dibromohexane. Après recristallisation dans l'isopropanol on obtient le dérivé du titre de pF = 253°C .

### EXEMPLE 15 : 1,1-(1,16-hexadécandiyl) bis-(4-méthyl 2-(1H) pyrimidine) dibromhydrate.

On opère dans les conditions habituelles avec du -1,16-dibromohexadécane et après recristallisation dans le mélange isopropanol/méthanol, on obtient le dérivé du titre de pF = 167/170°C.

En variante, ce produit est préparé comme suit :

Dans un erlenmeyer rodé de 100 ml, muni d'un réfrigérant et d'un agitateur magnétique, on introduit 2,11 g de 2-amino-4-méthylpyridine (3 équivalents) et 249 mg (0,65 mmole) de 1,16-dibromododécane dans 5 ml de butanone. Le mélange est chauffé à reflux 36 heures ; on évapore le solvant et on ajoute de l'isopropanol puis de l'éther, jusqu'à obtention d'un trouble. On laisse la solution à 4° pendant 2 jours. Le précipité obtenu est filtré et séché au dessicateur. On obtient ainsi un produit avec un rendement de 39 %.

RMN¹H dans DMSO-D₆ (δ en ppm) : 1,2 (m, 24H) ; 1,65 (m, 4H) ; 2,3 (s, 6H) ; 4,1 (t, 4H) ; 6,8 (m, 4H) ; 7,95 (d, 2H) ; 8,3 (s, 4H).

### EXEMPLE 16 : 1,1-(1,16-hexadécandiyl) bis-(4-éthyl 2-(1H) pyrimidine) dibromhydrate.

A partir de la 4-éthyl-2-aminopyridine décrite par Hansch et du 1,16-dibromohexadécane, on obtient le dérivé du titre de pF = 105/110°C.

En utilisant la méthode de l'exemple 1 et à partir des diverses diméthyl-2-aminopyridines synthétisées selon la littérature (Dornow, Rohe, Chem. Ber., 93, 1960, 1093/1097 ; Wentrup, Mayov, J. Amer. Chem. Soc., 97, 1975, 7468/7477 ; Rao Venkateswarlu, J. Heterocycl. Chem., 12, 1975, 731/732), on obtient les 1,1-(1,12-dodécanediyl) bis (diméthyl-substituées-2-(1H) pyridinimine) à l'état de dibromhydrates comme suit :

### EXEMPLE 17 : 1,1-(1,12-dodécanediyl) bis (3,4-diméthyl-2-(1H) pyridinimine).

### EXEMPLE 18 : 1,1-(1,12-dodécanediyl) bis (3,5-diméthyl-2-(1H) pyridinimine).

### EXEMPLE 19 : 1,1-(1,12-dodécanediyl) bis (3,6-diméthyl-2-(1H) pyridinimine)

### EXEMPLE 20 : 1,1-(1,12-dodécanediyl) bis (4,5-diméthyl-2-(1H) pyridinimine).

### EXEMPLE 21 : 1,1-(1,12-dodécanediyl) bis (4,6-diméthyl-2-(1H) pyridinimine).

### EXEMPLE 22 : 1,1-(1,12-dodécanediyl) bis (5,6-diméthyl-2-(1H) pyridinimine).

En utilisant la même méthode et à partir des éthyl-2-aminopyridines synthétisées, selon les procédés de la littérature comme Robison, J. Amer. Chem. Soc., 79, 1957, 2573/2576 ; Hansch, J. Org Chem., 23, 1958, 1924 ; Childress, J. Org. Chem., 23, 1958, 67 ; Yakhontov, Chem. Heterocycl. Compd, 3, 1967, 829/830, on obtient les 1,1-(1,12-dodécanediyl) bis (alkyl-substituées-2-(1H) pyridinimine)

### EXEMPLE 23 : 1,1-(1,12-dodécanediyl) bis (3-éthyl-2-(1H) pyridinimine).

### EXEMPLE 24 : 1,1-(1,12-dodécanediyl) bis (4-éthyl-2-(1H) pyridinimine).

### EXEMPLE 25 : 1,1-(1,12-dodécanediyl) bis (5-éthyl-2-(1H) pyridinimine).

### EXEMPLE 26 : 1,1-(1,12-dodécanediyl) bis (6-éthyl-2-(1H) pyridinimine).

### TESTS PHARMACOLOGIQUES :

- ACTIVITE ANTIPALUDEENNE *IN VITRO* VIS-A-VIS DE *PLASMODIUM FALCIPARUM :*
L'évaluation de l'activité antipaludéenne *in vitro* est réalisée selon la méthode de DESJARDINS, CANFIELD, HATNES et CHULAY (Antimicrob. Agents Chemother., 1979, 16, 710-718). Un composé conforme à l'invention est mis en contact pendant environ 48 heures avec les érythrocytes humains infectés par *Plasmodium falciparum.* Lorsqu'un précurseur radioactif d'acides nucléiques, l'hypoxanthine, est ajouté à la préparation, seuls les érythrocytes infectés par *Plasmodium* et non affectés par ledit composé incorporent ce précurseur. L'aptitude ou l'inaptitude à incorporer le précurseur reflète donc la viabilité des cellules impaludées. Les résultats sont présentés dans le Tableau I ci-après, sous forme d'IC 50 (ou DE 50) (concentration en composé capable d'inhiber *in vitro* la croissance des parasites de 50 %).
Les dérivés suivant les exemples précédents sont testés à trois concentrations 100, 33, 10 nanomolaires.
La vérification du mécanisme d'action est réalisée sur les divers dérivés selon l'invention par étude de l'interférence spécifique avec la biosynthèse des phospholipides et mesure de la biosynthèse des différentes biomolécules, acides nucléiques, protéines et phospholipides à partir de l'incorporation de précurseurs radiomarqués, (³H) hypoxanthine (cf. ci-dessus), (³H) isoleucine et (³H) choline. La spécificité au sein même du métabolisme phospholipidique est déterminée par comparaison avec l'effet sur l'incorporation de (³H) éthanolamine dans la phosphatidyléthanolamine (Ancelin M.L., Vialettes F. et Vial H.J., 1991, Anal. Biochem., 199, 203-209). Dans ces conditions, et parmi l'ensemble des dérivés décrits ci-dessus, c'est le composé de l'exemple 1 qui s'est révélé avoir la meilleure spécificité d'action vis-à-vis de la biosynthèse de phosphatidylcholine, par rapport aux biosynthèses des autres macromolécules (ac. nucléiques, protéines, autres phospholipides).
- ACTIVITE ANTIPALUDEENNE ET TOXICITE *IN VIVO :*
Cette activité est mesurée suivant le test décrit par PETERS W. (*Chemotherapy and Drug resistance in Malaria,* 1970). Un composé conforme à l'invention est administré pendant 4 jours consécutifs à des souris préalablement impaludées par *Plasmodium vinckei, petteri* ou *chabaudi.*
Ledit composé est dissous dans une solution aqueuse de NaCl à 0,9 % ou éventuellement dans une solution aqueuse de gomme arabique à 10 %. Les préparations sont ainsi administrées par voie intrapéritonéale ou par voie sous-cutanée à des souris Swiss mâles de poids moyen 30 à 40 g, qui ont été préalablement infectées par voie IV. avec *Plasmodium petterei* ou *P. chabaudi* (10⁶ cellules infectées). Le composé est administré 2 fois par jour pendant 4 jours consécutifs, la première injection étant pratiquée 2 heures après l'infestation et la deuxième, 10 heures après. La parasitémie est déterminée par un frottis, le jour suivant la fin du traitement.
Les résultats sont rassemblés dans le Tableau II, sous forme de DE 50 (dose de composé administré *in vivo* capable d'une inhibition de 50 % de la croissance des parasites). Ce Tableau II fait également apparaître la toxicité mesurée selon la même méthode, les animaux ayant reçu, dans les conditions d'administration utilisées pour les tests ci-dessus, 2 injections par jour pendant quatre jours (toxicité semichronique) ; les résultats sont exprimés sous forme de DL 50 (dose de composé entraînant la mort de 50 % des animaux).
- ACTIVITE ANTIBABESIA *IN VITRO :*
Le procédé de screening utilisé pour la série antimalaria et basé sur l'incorporation de (³H) hypoxanthine par le parasite est utilisable pour tester l'activité antimétabolite de tels composés sur *Babesia bovis.* Une relation étroite est trouvée entre le taux d'incorporation de (³H) hypoxanthine dans une mesure standard et le pourcentage de cellules parasitées, déterminé par examen microscopique. Les concentrations des composés provoquant une inhibition de 50 % de l'incorporation de (³H) hypoxanthine (ID50) sont résumées dans -le Tableau I ci-après.

**TABLEAU I**

| Composé | *Plasmodium* IC 50 *in vitro* rép.100 nM | *Plasmodium* IC 50 *in vitro* rép. à 33 nM | *Plasmodium* IC 50 *in vitro* rép. à 10 nM | *Plasmodium* IC 50 *in vitro* rép. <10nM | *Babesia bovis* -Activité à 10 mMol. |
|---|---|---|---|---|---|
| Exemple 1 | + | + | + | + | + |
| Exemple 2 | + | + | + | + | + |
| Exemple 3 | + | + | + | + | + |
| Exemple 4 | + | + | + | - | + |
| Exemple 5 | + | + | + | - | |
| Exemple 6 | + | + | + | - | + |
| Exemple 7 | + | + | + | - | - |
| Exemple 8 | + | + | + | - | + |
| Exemple 9 | + | + | + | - | - |
| Exemple 10 | + | + | + | - | + |
| Exemple 11 | + | + | + | - | + |
| Exemple 12 | + | - | | - | - |
| Exemple 13 | + | + | - | - | - |
| Exemple 14 | + | - | - | - | - |
| Exemple 15 | + | + | - | - | + |
| Exemple 16 | + | - | - | - | + |
| Exemple 17 | + | + | + | - | + |
| Exemple 18 | + | + | + | - | + |
| Exemple 19 | + | + | + | + | + |
| Exemple 20 | + | + | - | - | + |
| Exemple 21 | + | + | + | + | + |
| Exemple 22 | + | + | + | - | - |
| Exemple 23 | + | - | - | - | - |
| Exemple 24 | - | | - | - | - |
| Exemple 25 | + | + | - | - | - |
| Exemple 26 | + | + | + | - | - |

**TABLEAU II**

| Composé | Activité *in vitro* IC 50 nanoMolaire | Activité *in vivo* DE 50 mg/kg | Toxicité DL50 mg/kg | Toxicité DL50 mg/kg |
|---|---|---|---|---|
| | | I.P. | I.P. | Per Os |
| Exemple 1 | 0.5 | 1.2 | 30 | 365 |
| Exemple 2 | 1.8 | 3 | 20 | > 500 |
| Exemple 3 | 0.5 | 3 | 6.5 | >300 |
| Exemple 9 | 1.4 | 4 | 10 | >200 |
| Exemple 23 | 7.2 | > 5 | 23 | > 400 |
| Exemple 25 | 3.8 | > 5 | 7.5 | > 200 |

## Revendications

1. Bis-2-aminopyridine de formule générale (I), 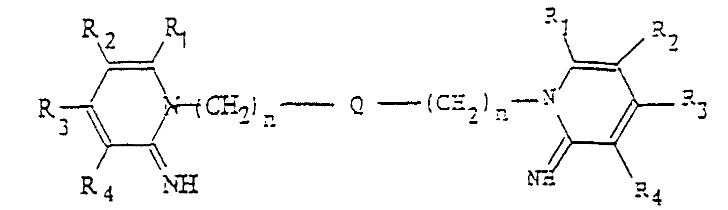 dans laquelle :
Q représente :
- un groupement alkyle, saturé ou insaturé, en C₆-C₂₀,
- un groupement aryle, éventuellement substitué, ou
- un groupement cycloalkyle, et
forme, entre les 2 noyaux pyridine et avec les groupements (CH₂)ₙ, un enchaînement hydrocarboné comprenant au total de 6 à 34 atomes de carbone,
n est un nombre compris entre 0 et 7,
R₁, R₂, R₃, R₄, identiques ou différents, représentent :
- un atome hydrogène,
- un groupement alkyle en C₁-C₆, linéaire ou ramifié, éventuellement substitué,
- un groupement aryle, éventuellement substitué par un ou plusieurs atomes d'halogène, ou des radicaux alkyle en C₁-C₆ ou alcoxy en C₁-C₆,
- un groupement benzyle,
- un groupement thiényle,
- un groupement furyle,
- un atome d'halogène,
- un groupement alcoxy ou benzyloxy
- un groupement trifluorométhyle,
- un groupement vinyle ou allyle,
à la condition qu'au moins l'un des radicaux R1, R2, R3 ou R4 ne soit pas un atome d'hydrogène lorsque Q représente un enchaînement (CH₂)ₘ avec m compris entre 6 et 20, ainsi que leurs sels d'addition.

2. Bis-2-aminopyridine, selon la revendication 1, caractérisées en ce que R₁, R₂, R₃ ou R₄ représentent un groupe méthyle, chloro, phényle, en positions 3, 4 et/ou 5 et Q représente un groupement alkyle en C₆-C₂₀, l'enchaînement hydrocarboné entre les deux noyaux pyridine, constituant un dodécane, un hexadécane, un octane ou un décane.

3. Procédé de préparation d'une bis-2-amino-pyridine de formula générale (I) selon la revendication 1 ou la revendication 2, caractérisé en ce que l'on fait réagir, en solution, un dérivé de formule (II) Hal-(CH₂)ₙ-Q-(CH₂)ₙ-Hal, dans laquelle Hal représente un atome d'halogène, n et Q ont la signification générale indiquée précédemment, avec un composé de formule (III) 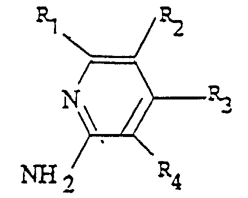 dans laquelle R₁, R₂, R₃ et R₄ ont la signification donnée précédemment,
pour obtenir le produit de formule générale (I), à l'état de dihalogénure correspondant que, si désiré, l'on basifie et l'on convertit en un autre sel d'acide.

4. Procédé selon la revendication 3, caractérisé en ce que le composé de formule II et le composé de formule III sont présents dans un rapport 1:2, le composé de formule II étant de préférence en léger excès.

5. Médicament, caractérisé en ce qu'il comprend au moins une bis-2-aminopyridine selon la revendication 1 ou la revendication 2.

6. Compositions pharmaceutiques, caractérisée en ce qu'elles comprennent comme constituant actif, une dose efficace d'au moins un produit de formule (I) selon la revendication 1 ou la revendication 2 ou d'un sel d'addition d'acide d'un tel composé avec, en plus des excipients et additifs convenables.

7. Bis-2-aminopyridine de formule générale (I) selon la revendication 1, dans laquelle R₁, R₂, R₃ et R₄ représentent des atomes d'hydrogène et Q représente un groupement alkyle en C₆-C₂₀ en tant que médicament.

8. Composition pharmaceutique pour combattre les parasitoses intra-érythrocytaires, caractérisée en ce qu'elle comprend une quantité active du composé selon la revendication 7, comme substance active.

9. Composition pharmaceutique pour combattre les parasitoses intra-érythrocytaires, caractérisée en ce qu'elle comprend une quantité active d'au moins un composé selon la revendication 1 ou la revendication 2.

## Patentansprüche

1. Bis-2-aminopyridin der allgemeinen Formel (I) 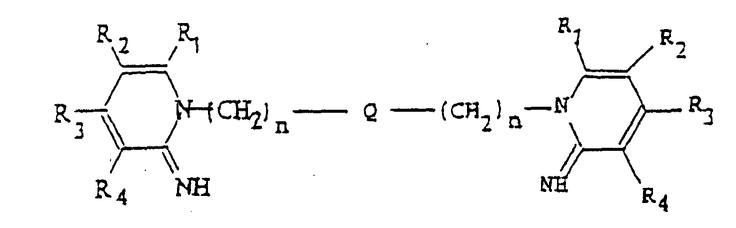 worin:
Q für
- eine gesältigte oder ungesältigte C₆-C₂₀-Alkylgruppe,
- eine gegebenenfalls substituierte Arylgruppe oder
- eine Cycloalkylgruppe steht, und
zwischen den 2 Pyridinringen und mit den (CH₂)ₙ-Gruppen eine Kohlenwasserstoffkette bildet, die insgesamt 6 bis 34 Kohlenstoffatome enthält,
n eine Zahl zwischen 0 und 7 ist,
R₁, R₂, R₃, R₄, die gleich oder verschieden sein können, für
- ein Wasserstoffatom,
- eine lineare oder verzweigte, gegebenenfalls substituierte C₁-C₆-Alkylgruppe,
- eine gegebenenfalls durch 1 oder mehrere Halogenatome oder C₁-C₆-Alkylreste oder C₁-C₆-Alkoxyreste substituierte Arylgruppe,
- eine Benzylgruppe,
- eine Thienylgruppe,
- eine Furylgruppe,
- ein Halogenatom,
- eine Alkoxy- oder Benzyloxygruppe,
- eine Trifluormethylgruppe,
- eine Vinyl- oder Allylgruppe
stehen,
mit der Maßgabe, daß mindestens einer der Reste R₁, R₂, R₃ oder R₄ nicht für ein Wasserstoffatom steht, wenn Q für eine (CH₂)ₘ-Kette steht, wobei m zwischen 6 und 20 liegt, sowie Additionssalze davon.

2. Bis-2-aminopyridin nach Anspruch 1, dadurch **gekennzeichnet**, daß R₁, R₂, R₃ oder R₄ für eine Methyl-, Chlor-, Phenylgruppe in den Positionen 3, 4 und/oder 5 stehen und Q für eine C₆-C₂₀-Alkylgruppierung steht, wobei die Kohlenwasserstoffkette zwischen den zwei Pyridinringen ein Dodecan, ein Hexadecan, ein Octan oder ein Decan ist.

3. Verfahren zur Herstellung eines Bis-2-aminopyridins der allgemeinen Formel (I) nach Anspruch 1 oder 2, dadurch **gekennzeichnet**, daß man in Lösung ein Derivat der Formel (II) Hal-(CH₂)ₙ-Q-(CH₂)ₙ-Hal, worin Hal für ein Halogenatom steht, n und Q die allgemeine oben angegebene Bedeutung haben, mit einer Verbindung der Formel (III) 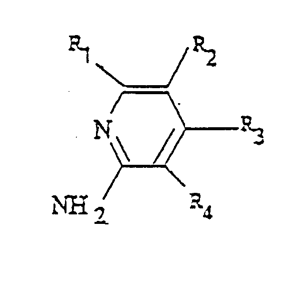 worin R₁, R₂, R₃ und R₄ die zuvor angegebene Bedeutung haben, umsetzt,
um eine Produkt der allgemeinen Formel (I) im Zustand des entsprechenden Dihalogenids zu erhalten, das man, falls gewünscht, basisch macht und in ein anderes Säuresalz umwandelt.

4. Verfahren nach Anspruch 3, dadurch **gekennzeichnet**, daß die Verbindung der Formel II und die Verbindung der Formel III im Verhältnis 1:2 vorliegen, wobei die Verbindung der Formel II vorzugsweise in geringem Überschuß vorliegt.

5. Medikament, dadurch **gekennzeichnet**, daß es mindestens ein Bis-2-aminopyridin nach Anspruch 1 oder 2 umfaßt.

6. Pharmazeutische Zusammensetzungen, dadurch **gekennzeichnet**, daß sie als aktiven Bestandteil eine wirksame Menge von mindestens einem Produkt der Formel (I) nach Anspruch 1 oder 2 oder einem Säureadditionssalz einer solchen Verbindung zusammen mit zusätzlichen geeigneten Träger- und Zusatzstoffen umfassen.

7. Bis-2-aminopyridin der allgemeinen Formel (I) nach Anspruch 1, worin R₁, R₂, R₃ und R₄ für Wasserstoffatome stehen und Q für eine C₆-C₂₀-Alkylgruppierung steht als Medikament.

8. Pharmazeutische Zusammensetzung zur Bekämpfung intraerythrozytärer Parasitosen, dadurch **gekennzeichnet**, daß sie eine aktive Menge der Verbindung nach Anspruch 7 als Wirkstoff umfaßt.

9. Pharmazeutische Zusammensetzung zur Bekämpfung intraerythrozytärer Parasitosen, dadurch **gekennzeichnet**, daß sie eine aktive Menge mindestens einer Verbindung nach Anspruch 1 oder 2 umfaßt.

## Claims

1. Bis(2-aminopyridine) of general formula (I), 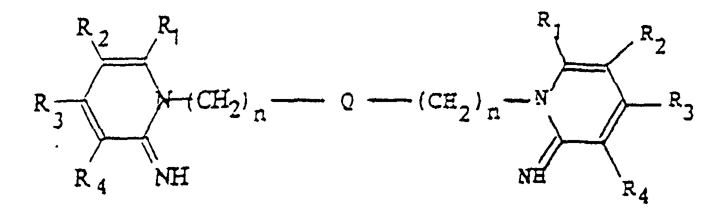 in which:
Q represents:
- a C₆-C₂₀ saturated or unsaturated alkyl group,
- an optionally substituted aryl group, or
- a cycloalkyl group, and
forms, between the 2 pyridine rings and with the groups (CH₂)ₙ, a hydrocarbon linkage comprising in total from 6 to 34 carbon atoms,
n is a number between 0 and 7,
R₁, R₂, R₃ and R₄, which may be identical or different, represent:
- a hydrogen atom,
- an optionally substituted, linear or branched C₁-C₆ alkyl group,
- an aryl group optionally substituted with one or more halogen atoms or C₁-C₆ alkyl or C₁-C₆ alkoxy radicals,
- a benzyl group,
- a thienyl group,
- a furyl group,
- a halogen atom,
- an alkoxy or benzyloxy group,
- a trifluoromethyl group,
- a vinyl or allyl group,
on condition that at least one of the radicals R₁, R₂, R₃ and R₄ is not a hydrogen atom when Q represents a linkage (CH₂)ₘ with m between 6 and 20, as well as their addition salts.

2. Bis(2-aminopyridine), according to Claim 1, characcerized in that R₁, R₂, R₃ or R₄ represents a methyl, chloro or phenyl group at positions 3, 4 and/or 5, and Q represencs a C₆-C₂₀ alkyl group, the hydrocarbon linkage between the two pyridine rings constituting a dodecane, a hexadecane, an octane or a decane.

3. Process for preparing a bis(2-aminopyridine) of general formula (I) according to Claim 1 or Claim 2, characterized in that a derivative of formula (II) Hal-(CH₂)ₙ-Q-(CH₂)ₙ-Hal, in which Hal represents a halogen atom and n and Q have the general meaning stated above, is reacted in solution with a compound of formula (III): 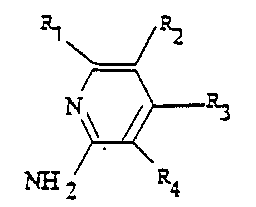 in which R₁, R₂, R₃ and R₄ have the meaning given above,
to obtain the product of general formula (I) in the state of the corresponding dihalide which, if desired, is alkalinized and converted to another salt of an acid.

4. Process according to Claim 3, characterized in that the compound of formula II and the compound of formula III are present in a 1:2 ratio, the compound of formula II preferably being in slight excess.

5. Medicinal product, characterized in that it comprises at least one bis(2-aminopyridine) according to Claim 1 or Claim 2.

6. Pharmaceutical compositions, characterized in that they comprise as active constituent an effective dose of at least one product of formula (I) according to Claim 1 or Claim 2 or of an addition salt with an acid of such a compound, with, in addition, appropriate excipients and additives.

7. Bis(2-aminopyridine) of general formula (I) according to Claim 1, in which R₁, R₂, R₃ and R₄ represent hydrogen atoms and Q represents a C₆-C₂₀ alkyl group, as a medicinal product.

8. Pharmaceutical composition for combating intraerythrocytic parasitoses, characterized in that it comprises an active amount of the compound according to Claim 7, as active substance.

9. Pharmaceutical composition for combating intraerythrocytic parasitoses, characterized in that it comprises an active amount of at least one compound according to Claim 1 or Claim 2.
